# EUROPEAN PATENT SPECIFICATION

(11) **EP 4 440 549 B1**
(45) Date of publication and mention of the grant of the patent: **12.11.2025**
(21) Application number: 22829712.3
(22) Date of filing: 01.12.2022
(51) Int. Cl.: A61K 9/08, A61K 47/14, A61K 47/26, A61K 47/44, A61P 27/02, A61K 31/506

(54) **OPHTHALMIC PHARMACEUTICAL COMPOSITIONS CONTAINING A NITRIC OXIDE-RELEASING PHOSPHODIESTERASE 5 INHIBITOR AND METHOD FOR THE PREPARATION THEREOF**
OPHTHALMISCHE PHARMAZEUTISCHE ZUSAMMENSETZUNGEN MIT EINEM STICKOXIDFREISETZENDEN PHOSPHODIESTERASE-5-HEMMER UND VERFAHREN ZUR HERSTELLUNG DAVON
COMPOSITIONS PHARMACEUTIQUES OPHTALMIQUES CONTENANT UN INHIBITEUR DE PHOSPHODIESTÉRASE 5 LIBÉRANT DE L'OXYDE NITRIQUE ET LEUR PROCÉDÉ DE PRÉPARATION

(30) Priority: 02.12.2021 EP 21211990
(43) Date of publication of application: 09.10.2024
(73) Proprietor: Nicox SA, 06410 Biot (FR)
(72) Inventor: ALMIRANTE, Nicoletta, 20155 MILANO (IT); BRAMBILLA, Stefania, 22046 MERONE (CO) (IT); GALLI, Corinna, 20010 MARCALLO CON CASONE (MI) (IT)
(74) Representative: Bianchetti & Minoja with Trevisan & Cuonzo IPS SRL
(86) International application number: PCT/EP2022/083986
(87) International publication number: WO 2023/099638

(56) References cited:
- WO-A1-2020/030489
- WO-A1-2021/156275

## Description

The present invention relates to aqueous ophthalmic formulations in the form of eye drops comprising [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate capable of maintaining physical and chemical stability of the active substance in the dosage form and a method for the preparation of such ophthalmic formulations.

[(2S)-1-(4-{[(3-Chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate is a nitric oxide releasing phosphodiesterase 5 inhibitor (NO-PDE5 inhibitor), hereafter referred to as Compound (I).

Compound (I) is disclosed in WO 2020/030489 that relates to NO-releasing PDE5 inhibitors and their use for the treatment of ocular diseases associated with elevated intraocular pressure, such as ocular hypertension and glaucoma, and for treating retinopathies. WO 2020/030489 does not disclose specific pharmaceutical formulations comprising said NO-releasing PDE5 inhibitors. WO 2021/156275 discloses NO-releasing PDE5 inhibitors and ophthalmic formulations thereof.

One obstacle to the preparation of an ophthalmic formulation comprising Compound (I) in the commonly used aqueous ophthalmic vehicles is its rather low aqueous solubility (0.013 mg/ml) being it largely lipophilic (clog D_{pH 6.7} = 5.76).

In an effort to enhance the solubility of Compound (I) in aqueous vehicle, 'salt forms' of Compound (I) were explored. However, the tested Compound (I) salts exhibited similar aqueous solubility as that of Compound (I) free base in the range of pH 6.0 - 7.0, which was the pH initially chosen for the ophthalmic formulation.

Additionally, making salts requires additional processing steps which increase costs of synthesis; furthermore, it decreases the overall reaction yield.

Therefore, there is a need for an aqueous formulation of Compound (I) containing therapeutically effective amounts of the active ingredient, is easily manufactured, has a long shelf life under normal handling conditions, and is well tolerated by the eye.

The present invention provides an ophthalmic composition in the form of aqueous solution comprising the following ingredients:
(i) [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate (Compound (I)) as the active ingredient,
(ii) a surfactant component consisting of a mixture of two non-ionic surfactants selected from the following: Macrogolglycerol ricinoleate (Kolliphor^{®} EL), Macrogol stearate 40 (Myrj^{™} S40), and Polyoxyethylenesorbitan monooleate (Tween^{®} 80),
(iii) a co-solvent selected from the class of polyols which also serves as an osmolality adjuster;
(iv) a buffer system to adjust the pH over a range of 4.5 to 7.5;
(v) water for injection or purified water;

The ophthalmic composition of the invention is in the form of eye drops for topical ocular administration.

The inventors have found that the combination of the surfactant component and the presence of a polyol are able to enhance the solubility of Compound (I) in aqueous vehicle and the resulting clear ophthalmic formulation is largely stable without insoluble deposit formation over time. Furthermore, when tested in animals it resulted well tolerated.

An embodiment of the invention provides an ophthalmic formulation comprising:
(i) 0.8% to 1.8% w/w of [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate or any specific value within said range;
(ii) a surfactant component selected from the following mixtures:
   - 4.0% to 7.5% w/w Macrogolglycerol ricinoleate and 1.0% to 6.0% w/w Macrogol stearate 40,
   - 4.0% to 7.5% w/w Macrogolglycerol ricinoleate and 1.0% to 7.0% w/w Polyoxyethylenesorbitan monooleate, or
   - 1.0% to 6.0% w/w Macrogol stearate 40 and 1.0% to 7.0% w/w Polyoxyethylenesorbitan monooleate;
(iii) 1.0% to 5.0% w/w of a co-solvent selected from the class of polyols;
(iv) a buffer system to adjust the pH in a range of 4.5 to 7.5;
(v) water for injection or purified water q.s. to 100% w/w,
and wherein the osmolality of the ophthalmic formulations is 240 to 400 mOsm/kg, preferably 240 to 380 mOsm/kg.

Another embodiment of the invention provides an ophthalmic formulation comprising:
(i) 1.0% to 1.8% w/w of [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate or any specific value within said range;
(ii) a surfactant component selected from the following mixtures:
   - 5.0% to 7.5% w/w Macrogolglycerol ricinoleate and 3.0% to 5.0% w/w Macrogol stearate 40,
   - 5.0% to 7.5% w/w of Macrogolglycerol ricinoleate and 2.0% to 5.0% w/w Polyoxyethylenesorbitan monooleate, or
   - 3.0% to 5.0% w/w Macrogol stearate 40 and 2.0% to 5.0% w/w Polyoxyethylenesorbitan monooleate;
(iii) 1.0% to 4.0% w/w Polyethylene glycol 400;
(iv) a buffer system to adjust the pH to 4.5 to 7.5;
(v) water for injection or purified water q.s. to 100% w/w;

and further comprising 0.01% to 0.10% w/w EDTA and 0.01% to 0.02% w/w benzalkonium chloride, and
wherein the osmolality of the ophthalmic formulations is 240 to 400 mOsm/kg, preferably 240 to 380 mOsm/kg.

An embodiment of the invention provides an ophthalmic formulation comprising:
(i) 1.0% to 1.5% w/w of [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate or any specific value within said range;
(ii) a surfactant component selected from the following mixtures:
   - 5.0% to 7.5% w/w Macrogolglycerol ricinoleate and 3.0% to 5.0% w/w Macrogol stearate 40,
   - 5.0% to 7.5% w/w Macrogolglycerol ricinoleate and 2.0% to 5.0% w/w Polyoxyethylenesorbitan monooleate, or
   - 3.0% to 5.0% w/w Macrogol stearate 40 and 2.0% to 5.0% w/w Polyoxyethylenesorbitan monooleate;
(iii) 1.0% to 4.0% w/w of a co-solvent selected from the class of polyols;
(iv) a buffer system to adjust the pH over a range of 4.5 to 7.5;
(v) water for injection or purified water q.s. to 100% w/w, and
wherein the osmolality of the ophthalmic formulations is 240 to 400 mOsm/kg, preferably 240 to 380 mOsm/kg.

In the formulations of the present invention the co-solvent of the class of polyols is preferably selected from mannitol, glycerol, sorbitol or polyethylene glycols; most preferably the co-solvent is Polyethylene glycol 400.

In the formulations of the present invention, the buffer system is preferably selected from the following: boric acid and disodium hydrogen phosphate heptahydrate, disodium hydrogen phosphate heptahydrate and sodium dihydrogen phosphate dihydrate, disodium hydrogen phosphate heptahydrate and citric acid, trisodium citrate dihydrate and citric acid monohydrate, trisodium citrate dihydrate and boric acid, boric acid. More preferably the buffer system is a mixture of boric acid and disodium hydrogen phosphate heptahydrate or citric acid monohydrate and trisodium citrate dehydrate or trisodium citrate dihydrate and boric acid or boric acid.

Optionally the ophthalmic composition further comprises other excipients such as a chelating agent and an antimicrobial preservative.

Preferably, the chelating agent is ethylenediaminetetraacetic acid (EDTA) that is present in the formulation in an amount from 0.01% to 0.2% w/w. Within the terms of the present invention, EDTA relates to ethylenediamine tetraacetic acid itself as well to its salts, namely e.g. disodium and / or potassium salts.

The antimicrobial preservative may be selected from quaternary ammonium compounds such as benzalkonium chloride (BAK), polyquaternium-1 (PQ-1), benzethonium chloride, potassium sorbate or sorbic acid or a mixture thereof, preferably the antimicrobial preservative is benzalkonium chloride.

Benzalkonium chloride is better described as: N-benzyl-N-(C₈-C₁₈alkyl)-N,N-dimethylammonium chloride. Preferably, the antimicrobial preservative is benzalkonium chloride. Benzalkonium chloride is present in the solution in an amount from 0.01% to 0.02% w/w.

Optionally hydrochloric acid and / or sodium hydroxide may be added to ophthalmic composition to adjust the pH of the final formulation to a pH over a range of 4.5 to 7.5.

Other optional excipients that may be included in the ophthalmic formulation are: tonicity adjusting agents such as glycerol, sorbitol, mannitol, dextrose, sodium or potassium chloride, viscosity enhancing agents such as methyl cellulose, hydroxypropyl methylcellulose, antioxidants or a mixture thereof.

A preferred embodiment of the invention provides an ophthalmic formulation consisting essentially of:
(i) 1.0% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 5.0% w/w Macrogolglycerol ricinoleate and 3.0% w/w Macrogol stearate 40;
(iii) 2.8% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride and HCl or NaOH to adjust the pH of the final solution to 6.5 - 6.9.

Another preferred embodiment of the invention provides an ophthalmic formulation consisting essentially of:
(i) 1.3% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Macrogol stearate 40;
(iii) 2.8% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride, and HCl or NaOH to adjust pH to 6.5 - 6.9.

Another preferred embodiment of the invention provides an ophthalmic formulation consisting essentially of:
(i) 1.4% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Macrogol stearate 40;
(iii) 4.0% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride, and HCl or NaOH to adjust pH to 6.5 - 6.9.

Another preferred embodiment of the invention provides an ophthalmic formulation consisting essentially of:
(i) 1.8% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride, and HCl or NaOH to adjust pH to 6.5 - 6.9.

Another preferred embodiment of the invention provides an ophthalmic formulation consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride.

Another preferred embodiment of the invention provides an ophthalmic formulation consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate and 0.73% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

Another preferred embodiment of the invention provides an ophthalmic formulation consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.003% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

Another preferred embodiment of the invention provides an ophthalmic formulation consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate, 0.73% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

Another preferred embodiment of the invention provides an ophthalmic formulation consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate, 0.73% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

The percentages of the components of the ophthalmic formulations of the invention are expressed with respect to the total weight of the formulation.

Another embodiment of the present invention provides the use of the ophthalmic composition of the invention for treating glaucoma, ocular hypertension, pathological conditions associated with elevated intraocular pressure or retinopathies.

The topical ophthalmic formulations of the present invention can be prepared using a process that comprises the following steps:
1) adding under stirring into the preparation vessel:
   - two of the following non-ionic surfactants: Macrogolglycerol ricinoleate (Kolliphor^{®} EL), Macrogol stearate 40 (Myrj^{™} S40) and Polyoxyethylenesorbitan monooleate (Tween^{®} 80),
   - a co-solvent,
   - at least 80% of the final weight of water for injection or purified water,
   - optionally an antimicrobial preservative,
   and stirring the mixture to obtain a solution;
2) adding a buffer system and stirring the mixture until complete solubilization, measuring the pH and optionally adjusting the pH between 4.5 to 7.5;
3) heating up the solution at 65°C, then adding [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate (Compound (I)) and stirring the mixture until dissolution of Compound (I);
4) cooling down the solution to room temperature (RT) under stirring;
5) optionally adding a chelating agent and stirring the mixture for complete solubilization;
6) optionally, adjusting the pH value to 4.5-7.5, preferably to pH 6.5 - 6.9;
7) adjusting the final weight of the solution with water for injection or purified water;
8) checking and adjusting the pH, if necessary, to 4.5-7.5, preferably to pH 6.5 - 6.9;
9) sterile filtering the bulk solution using a suitable filter with a nominal pore size of 0.2 µm or lower.

In particular, the topical ophthalmic formulations of the present invention can be prepared using a process that comprises the following steps:
1) adding under stirring into the preparation vessel:
   - two of the following non-ionic surfactants: Macrogolglycerol ricinoleate (Kolliphor^{®} EL), Macrogol stearate 40 (Myrj^{™} S40) and Polyoxyethylenesorbitan monooleate (Tween^{®} 80),
   - a co-solvent such as Polyethylene glycol 400 (Kollisolv^{®} PEG E 400),
   - 80% to 90% of the final weight of water for injection or purified water,
   - an antimicrobial preservative such as benzalkonium chloride,
   and stirring the mixture to obtain a solution;
2) adding a buffer system such as boric acid (H₃BO₃) and disodium hydrogen phosphate heptahydrate (Na₂HPO₄·7H₂O) and stirring the mixture until complete solubilization, measuring the pH and optionally adjusting the pH with HCl or NaOH between 4.5 to 7.5;
3) heating up the solution at 65°C, then adding [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate (Compound (I)) and stirring the mixture until dissolution of Compound (I);
4) cooling down the solution to room temperature (RT) under stirring;
5) adding a chelating agent such as disodium ethylenediaminetetraacetate dihydrate (EDTA) and stirring the mixture for complete solubilization;
6) optionally, adjusting the pH value to 4.5-7.5, preferably 6.5 - 6.9 by HCl and/or NaOH;
7) adjusting the final weight of the solution with water for injection or purified water;
8) checking and adjusting the pH, if necessary, to 4.5-7.5, preferably 6.5 - 6.9 by adding NaOH and/or HCl;
9) sterile filtering the bulk solution using a suitable filter with a nominal pore size of 0.2 µm or lower.

### EXAMPLES

[(2S)-1-(4-{[(3-chloro-4-methoxyphenyl)methyl]amino}-5-{[(pyrimidin-2-yl)methyl] carbamoyl}pyrimidin-2-yl)pyrrolidin-2-yl]methyl 6-(nitrooxy)hexanoate (Compound (I)) was prepared according to the method of synthesis disclosed in WO 2020/030489. Macrogolglycerol ricinoleate (Kolliphor^{®} EL), Macrogol stearate 40 (Myrj^{™} S40), Polyoxyethylenesorbitan monooleate (Tween^{®} 80) and Polyethylene glycol 400 (Kollisolv^{®} PEG E 400) are commercially available. The surfactants and co-solvents conform to the requirements of the European and US Pharmacopoeias.

The percentages of the components of the ophthalmic formulation are expressed with respect to the total weight of the formulation (%w/w).

Q.s. (quantum satis) means "Add as much of an ingredient as is needed to achieve the desired result".

### Example 1

This example illustrates the preparation of an ophthalmic formulation of the invention containing 1.0% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

5.0 g of Macrogolglycerol ricinoleate (Kolliphor^{®} EL), 3.0 g of Macrogol stearate 40 (Myrj^{™} S40), 2.8 g of Polyethylene glycol 400 (Kollisolv^{®} PEG 400), 0.01 g of benzalkonium chloride, and 69.9 g of water for injection were weighted into the preparation vessel. The mixture was stirred using a magnetic stir-plate until all components were dissolved and the achieved solution appeared to be slightly yellow or colorless and transparent. 0.19 g of boric acid (H₃BO₃) and 0.51 g of disodium hydrogen phosphate heptahydrate (Na₂HPO₄7H₂O) were added and the mixture was stirred until complete dissolution. The system was then heated up to 65°C before adding 1 g of Compound (I) and stirring for 30 minutes. The solution was then cooled down to room temperature (RT) under stirring. 0.10 g of disodium ethylenediaminetetraacetate dihydrate (EDTA) was added and further stirred to have a clear solution. The pH of the formulation was then measured and adjusted to 6.5 - 6.9 by adding an aqueous solution of HCl (0.5M). The remaining water for injection to reach the final weight of 100 g was added and the pH was measured again. The bulk formulation was then filtered under sterile conditions by means of suitable filter with a nominal pore size of 0.2 µm.

### Example 2

This example illustrates the preparation of an ophthalmic formulation of the invention containing 1.3% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy) hexanoate (Compound (I)).

7.5 g of Macrogolglycerol ricinoleate (Kolliphor^{®} EL), 5.0 g of Macrogol stearate 40 (Myrj^{™} S40), 2.8 g of Polyethylene glycol 400 (Kollisolv^{®} PEG 400), 0.01 g of benzalkonium chloride, and 66.1 g of water for injection were weighted into the preparation vessel. The mixture was stirred using a magnetic stir-plate until all components were dissolved and the resulting solution appeared as slightly yellow or colorless and transparent. 0.19 g of boric acid (H₃BO₃) and 0.51 g of disodium hydrogen phosphate heptahydrate (Na₂HPO₄·7H₂O) were added and the mixture was stirred until complete dissolution of all ingredients. The system was then heated up to 65°C before adding 1.3 g of Compound (I) followed by additional 30 minutes stirring. The solution was then cooled down to room temperature (RT) under stirring. 0.10 g of disodium ethylenediaminetetraacetate dihydrate was added and the solution was stirred until complete solubilization. The pH of the resulting formulation was measured and adjusted to 6.5 - 6.9 by adding an aqueous solution of HCl (0.5M). The remaining water for injection to reach the final weight of 100 g was added the pH was measured again. The bulk formulation was finally filtered under sterile conditions by means of suitable filter with a nominal pore size of 0.2 µm.

### Example 3

This example illustrates the preparation of an ophthalmic formulation of the invention containing 1.4% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

7.5 g of Macrogolglycerol ricinoleate (Kolliphor^{®} EL), 5.0 g of Macrogol stearate 40 (Myrj^{™} S40), 4.0 g of Polyethylene glycol 400 (Kollisolv^{®} PEG 400), 0.01 g of benzalkonium chloride, and 65.0 g of water for injection were weighted into the preparation vessel. The mixture was stirred using a magnetic stir-plate until all components were dissolved and the achieved solution appeared to be slightly yellow or colorless and transparent. 0.19 g of boric acid (H₃BO₃) and 0.51 g of disodium hydrogen phosphate heptahydrate (Na₂HPO₄·7H₂O) were added and the mixture was stirred until complete dissolution. The system was then heated up to 65°C before adding 1.4 g of Compound (I) and stirring for 30 minutes. The solution was then cooled down to room temperature (RT) under stirring. 0.10 g of disodium ethylenediaminetetraacetate dihydrate was added and the solution was stirred until complete solubilization. The pH of the achieved formulation was measured and adjusted to 6.5 - 6.9 by adding an aqueous solution of HCl (0.5M). The remaining water for injection to reach the final weight of 100 g was added the pH was measured again. The bulk formulation was then filtered under sterile conditions by means of suitable filter with a nominal pore size of 0.2 µm.

### Example 4

This example illustrates the preparation of an ophthalmic formulation of the invention containing 1.8% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy) hexanoate (Compound (I)).

7.5 g of Macrogolglycerol ricinoleate (Kolliphor^{®} EL), 5.0 g of Polyoxyethylenesorbitan monooleate (Tween^{®} 80), 1.0 g of Polyethylene glycol 400 (Kollisolv^{®} PEG 400), 0.01 g of benzalkonium chloride, and 67.1 g of water for injection were weighted into the preparation vessel. The mixture was stirred using a magnetic stir-plate until all components were dissolved and the achieved solution appeared to be slightly yellow or colorless and transparent. 0.19 g of boric acid (H₃BO₃) and 0.51 g of disodium hydrogen phosphate heptahydrate (Na₂HPO₄·7H₂O) were added and the mixture was stirred until complete dissolution. The system was then heated up to 65°C before adding 1.8 g of Compound (I) and stirring for 30 minutes. The solution was then cooled down to room temperature (RT) under stirring. 0.10 g of disodium ethylenediaminetetraacetate dihydrate was added and the solution was stirred until complete solubilization. The pH of the achieved formulation was measured and adjusted to 6.5 - 6.9 by adding an aqueous solution of HCl (0.5M). The remaining water for injection to reach the final weight of 100 g was added and pH was measured again. The bulk formulation was then filtered under sterile conditions by means of suitable filter with a nominal pore size of 0.2 µm.

### Example 5

### Stability Studies of formulation described in Example 1

The ophthalmic formulation of Example 1 was stored at 25°C for stability analysis. The samples were analyzed at different time points up to 37 weeks. The results are reported in Table 1.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 1: Stability data of ophthalmic formulation of Example 1** | | | | | | | | | |
|---|---|---|---|---|---|---|---|---|---|
| **Time (weeks)** | t0 (release) | t4 | t8 | t13 | t17 | t21 | t25 | t27 | t37 |
| pH | 6.6 | 6.6 | 6.5 | 6.6 | 6.5 | 6.4 | 6.6 | 6.5 | ND |
| Compound (I) Purity (area%) | 96.6 | 96.3 | 96.1 | 95.7 | 95.3 | 95.5 | 95.1 | 94.8 | 94.2 |
| Compound (I) Quantification (%w/w) | 1.1 | 1.1 | 1.2 | 1.1 | 1.1 | 1.1 | 1.1 | 1.1 | 1.2 |
| Osmolality (mOsm/kg) | ND | ND | 322 | ND | 327 | ND | ND | ND | ND |
| Macrosopic appearance | At each time point the formulations appeared colourless, free of visible particles | | | | | | | | |

### Example 6

### Stability Studies of formulation described in Example 2

The ophthalmic formulation of Example 2 was stored at 25°C for stability analysis. The samples were analyzed at different time points up to 8 weeks. The results are reported in Table 2.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 2: Stability data of formulation of Example 2** | | | | |
|---|---|---|---|---|
| Time (weeks) | t0 (Release) | t2 | t4 | t8 |
| pH | 6.7 | 6.6 | 6.8 | 6.5 |
| Compound (I) Purity (area%) | 96.8 | 96.4 | 96.5 | 96.2 |
| Compound (I) Quantification (%w/w) | 1.4 | 1.4 | 1.3 | 1.3 |
| Macroscopic appearance | At each time point the formulations appeared slightly yellow, free of visible particles | | | |

### Example 7

### Stability Studies of formulation described in Example 3

The ophthalmic formulation of Example 3 was stored at 25°C for stability analysis. The samples were analyzed at different time points up to 8 weeks. The results are reported in Table 3.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 3: Stability data of formulation of Example 3** | | | | |
|---|---|---|---|---|
| **Time (weeks)** | t0 (Release) | t2 | t4 | t8 |
| pH | 6.7 | 6.7 | 6.9 | 6.6 |
| Compound (I) Purity (area%) | 96.7 | 96.4 | 96.4 | 96.2 |
| Compound (I) Quantification (%w/w) | 1.5 | 1.5 | 1.4 | 1.4 |
| Macroscopic appearance | At each time point the formulations appeared slightly yellow, free of visible particles | | | |

### Example 8

### Tolerability study

The ophthalmic formulation of Example 1 was studied in non-human primates as well as in New Zealand White rabbits. Specifically, the ophthalmic formulation of Example 1 or the correspondent aqueous vehicle (5.0% w/w Macrogolglycerol ricinoleate and 3.0% w/w Macrogol stearate 40, 2.8% w/w Polyethylene glycol 400, 0.19% w/w boric acid, 0.51% w/w disodium hydrogen phosphate heptahydrate, 0.1% w/w disodium ethylenediaminetetraacetate dihydrate, 0.01% w/w benzalkonium chloride, HCl and water for injection q.s. to 100% w/w, pH 6.5 - 6.9) were administered acutely in non-human primates and repeatedly twice daily for up to 4 weeks in New Zealand White rabbits. In all the testing groups the administration of the ophthalmic formulation as well as that of the respective vehicle did not result in appreciable signs of discomfort as determined by visual inspection.

### Example 9

This example illustrates the preparation of an ophthalmic formulation of the invention containing 1.7% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

Macrogolglycerol ricinoleate (Kolliphor^{®} EL) 1.5 g, Polyoxyethylenesorbitan monooleate (Tween^{®} 80) 1.0 g, Polyethylene glycol 400 (Kollisolv^{®} PEG E 400) 200 mg, benzalkonium chloride 2 mg and water for injection 13.5 g were weighted into the preparation vessel. The mixture was stirred using a magnetic stir-plate until obtaining a slightly yellow or colorless and transparent solution. Boric acid (H₃BO₃) 38 mg was added and the mixture was stirred until complete dissolution. The mixture was heated up to 65°C, then Compound (I) 340 mg was added and the mixture was stirred for 30 minutes. The solution was cooled down to room temperature (RT) under stirring, disodium ethylenediaminetetraacetate dihydrate 20 mg was added and the mixture was stirred until complete solubilization. The pH of the formulation was 5.6. Water for injection was added to adjust the final weight to 20 g. The resulting mixture was a solution. The bulk solution was then filtered under sterile conditions by means of suitable filter with a nominal pore size of 0.2 µm.

### Example 10

This example illustrates the preparation of an ophthalmic formulation of the invention containing 1.7% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

Macrogolglycerol ricinoleate (Kolliphor^{®} EL) 1.5 g, Polyoxyethylenesorbitan monooleate (Tween^{®} 80) 1.0 g, Polyethylene glycol 400 (Kollisolv^{®} PEG E 400) 200 mg, benzalkonium chloride 2 mg, water for injection 13.5 g were weighted into the preparation vessel. The mixture was stirred using a magnetic stir-plate until after a slightly yellow or colorless and transparent solution was obtained. Citric acid monohydrate (C₆H₈O₇·H₂O, 22 mg) and trisodium citrate dihydrate (C₆H₅Na₃O₇·2H₂O), 146 mg) were added and the mixture was stirred until after all ingredients were dissolved. The mixture was heated up to 65°C and then Compound (I) 340 mg was added followed by 30 minutes continuous stirring. The solution was then cooled down to room temperature (RT) under stirring. Disodium ethylenediaminetetraacetate dihydrate 20 mg was added and the solution was stirred to obtain a clear solution. The pH of the formulation was 5.8. Water for injection was added to adjust the final weight to 20 g. The resulting mixture was a solution. The bulk solution obtained was finally filtered under sterile conditions by means of a suitable filter with a nominal pore size of 0.2 µm.

### Example 11

This example illustrates the preparation of an ophthalmic formulation of the invention containing 1.7% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

Macrogolglycerol ricinoleate (Kolliphor^{®} EL) 1.5 g, Polyoxyethylenesorbitan monooleate (Tween^{®} 80) 1.0 g, Polyethylene glycol 400 (Kollisolv^{®} PEG E 400) 200 mg, benzalkonium chloride 2 mg and water for injection 13.5 g were weighted into the preparation vessel. The mixture was stirred using a magnetic stir-plate to obtain a slightly yellow or colorless and transparent solution. Boric acid (H₃BO₃), 38 mg and trisodium citrate dihydrate (C₆H₅Na₃O₇·2H₂O) 0.6 mg were added and the mixture was stirred until complete dissolution. The mixture was heated up to 65°C, Compound (I) 340 mg was added and the resulting mixture stirred for 30 additional minutes. The solution was cooled down to room temperature (RT) under stirring. Disodium ethylenediaminetetraacetate dihydrate 20 mg was added and the mixture stirred until all ingredients were completely dissolved. The pH was 5.6. Water for injection was added to adjust the final weight to 20 g. The resulting mixture was a solution. The resulting bulk solution was later filtered under sterile conditions by means of a suitable filter with a nominal pore size of 0.2 µm.

### Example 12

This example illustrates the preparation of an ophthalmic formulation of the invention containing 1.7% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

Macrogolglycerol ricinoleate (Kolliphor^{®} EL) 750 mg, Polyoxyethylenesorbitan monooleate (Tween^{®} 80) 500 mg, Polyethylene glycol 400 (Kollisolv^{®} PEG E 400) 100 mg, benzalkonium chloride 1 mg, and water for injection 6.7 g were weighted into the preparation vessel. The mixture was stirred using a magnetic stir-plate until obtaining a slightly yellow or colorless and transparent solution. Citric acid monohydrate (C₆H₈O₇·H₂O) 11 mg and trisodium citrate dihydrate (C₆H₅Na₃O₇·2H₂O) 73 mg were added and the mixture was stirred until complete dissolution. The pH was adjusted to 5.0 by adding HCl 0.5M dropwise. The system was then heated up to 65°C, Compound (I) 170 mg was added and the mixture was stirred for 30 minutes. The solution was cooled down to room temperature (RT) under stirring. Disodium ethylenediaminetetraacetate dehydrate 10 mg was added and the mixture was stirred until complete solubilization. Water for injection was added to adjust the final weight to 10 g. The resulting mixture was a solution. The bulk solution was then filtered under sterile conditions by means of suitable filter with a nominal pore size of 0.2 µm.

### Example 13

This example illustrates the preparation of an ophthalmic formulation of the invention containing 1.7% w/w of [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)).

Macrogolglycerol ricinoleate (Kolliphor^{®} EL) 750 mg, Polyoxyethylenesorbitan monooleate (Tween^{®} 80) 500 mg, Polyethylene glycol 400 (Kollisolv^{®} PEG E 400) 100 mg, benzalkonium chloride 1 mg and water for injection 6.7 g were weighted into the preparation vessel. The mixture was stirred using a magnetic stir-plate until obtaining a slightly yellow or colorless and transparent solution. Citric acid monohydrate (C₆H₈O₇·H₂O) 11 mg and trisodium citrate dihydrate (C₆H₅Na₃O₇·2H₂O) 73 mg were added and the mixture was stirred until complete dissolution. The pH was adjusted to 4.5 by adding HCl 0.5M dropwise. The mixture was heated up to 65°C, Compound (I) 170 mg was added and the mixture was stirred for 30 minutes. The solution was cooled down to room temperature (RT) under stirring. Disodium ethylenediaminetetraacetate dihydrate 10 mg was added and the mixture was stirred until complete solubilization. Water for injection was added to adjust the final weight to 10 g. The resulting mixture was a solution. The bulk solution was then filtered under sterile conditions by means of suitable filter with a nominal pore size of 0.2 µm.

### Example 14

### Stability studies of formulation described in Example 4

The ophthalmic formulation of Example 4 was stored at 25°C for stability analysis. The samples were analyzed at t0 and up to 2 weeks. The results are reported in Table 4.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 4: Stability data of formulation of Example 4** | | |
|---|---|---|
| **Time (weeks)** | t0 (Release) | t2 |
| pH | 6.8 | 6.9 |
| Compound (I) Purity (area%) | 96.1 | 96.1 |
| Compound (I) Quantification (%w/w) | 1.8 | 1.8 |
| Macroscopic appearance | At each time point the formulations appeared slightly yellow, free of visible particles | |

### Example 15

### Stability Studies of formulation described in Example 9

The ophthalmic formulation of Example 9 was stored at 25°C for stability analysis. The samples were analyzed at different time points up to 14 weeks. The results are reported in Table 5.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 5: Stability data of formulation of Example 9** | | | | | |
|---|---|---|---|---|---|
| **Time (weeks)** | t0 (Release) | t2 | t4 | t8 | t14 |
| pH | 5.6 | 5.5 | 5.4 | 5.5 | 5.5 |
| Compound (I) Purity (area%) | 96.2 | 96.4 | 95.7 | 95.2 | 94.8 |
| Compound (I) Quantification (%w/w) | 1.7 | 1.7 | 1.8 | 1.7 | 1.7 |
| Macroscopic appearance | At each time point the formulations appeared slightly yellow, free of visible particles | | | | |

### Example 16

### Stability Studies of formulation described in Example 10

The ophthalmic formulation of Example 10 was stored at 25°C for stability analysis. The samples were analyzed at different time points up to 8 weeks. The results are reported in Table 6.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 6: Stability data of formulation of Example 10** | | | | |
|---|---|---|---|---|
| **Time (weeks)** | t0 (Release) | t2 | t4 | t8 |
| pH | 5.8 | 5.9 | 5.8 | 5.7 |
| Compound (I) Purity (area%) | 97.1 | 95.8 | 95.9 | 95.5 |
| Compound (I) Quantification (%w/w) | 1.7 | 1.8 | 1.8 | 1.8 |
| Macroscopic appearance | At each time point the formulations appeared slightly yellow, free of visible particles | | | |

### Example 17

### Stability Studies of formulation described in Example 11

The ophthalmic formulation of Example 11 was stored at 25°C for stability analysis. The samples were analyzed at different time points up to 8 weeks. The results are reported in Table 7.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 7: Stability data of formulation of Example 11** | | | | |
|---|---|---|---|---|
| **Time (weeks)** | t0 (Release) | t2 | t4 | t8 |
| pH | 5.6 | 5.6 | 5.5 | 5.5 |
| Compound (I) Purity (area%) | 97.2 | 95.9 | 95.9 | 95.5 |
| Compound (I) Quantification (%w/w) | 1.7 | 1.8 | 1.8 | 1.7 |
| Macroscopic appearance | At each time point the formulation appeared as slightly yellow solution, free of visible particles | | | |

### Example 18

### Stability Studies of formulation described in Example 12

The ophthalmic formulation of Example 12 was stored at 25°C for stability analysis. The samples were analyzed at different time points up to 15 weeks. The results are reported in Table 8.

The amount of Compound (I) was determined with an HPLC equipped with an UV detector and using a mixture of two mobile phases (A: ammonium acetate 10mM adjusted to pH 4.55 by adding acetic acid 50%; B: methanol).

| Table 8: Stability data of formulation of Example 12 | | | | | |
|---|---|---|---|---|---|
| **Time** (weeks) | t0 **(Release)** | t2 | t4 | t8 | t15 |
| pH | 5.0 | 5.2 | 5.2 | 5.1 | 4.8 |
| Compound (I) Purity (area%) | 96.3 | 95.6 | 95.0 | 94.3 | 93.4 |
| Compound (I) Quantification (%w/w) | 1.8 | 1.7 | 1.7 | 1.7 | 1.6 |
| Macroscopic appearance | At each time point the formulations appeared slightly yellow, free of visible particles | | | | |

### Example 19

### Stability Studies of formulation described in Example 13

The ophthalmic formulation of Example 13 was stored at 25°C for stability analysis. The samples were analyzed at different time points up to 15 weeks. The results are reported in Table 9.

The amount of Compound (I) was determined with an HPLC equipped with an UV detector and using a mixture of two mobile phases (A: ammonium acetate 10mM adjusted to pH 4.55 by adding acetic acid 50%; B: methanol).

| **Table 9: Stability data of formulation of Example 13** | | | | | |
|---|---|---|---|---|---|
| **Time (weeks)** | t0 (Release) | t2 | t4 | t8 | t15 |
| pH | 4.5 | 4.6 | 4.6 | 4.5 | 4.3 |
| Compound (I) Purity (area%) | 96.2 | 95.3 | 94.1 | 92.5 | 90.4 |
| Compound (I) Quantification (%w/w) | 1.8 | 1.7 | 1.7 | 1.6 | 1.5 |
| Macroscopic appearance | At each time point the formulations appeared slightly yellow, free of visible particles | | | | |

The stability data reported in Tables 1 to 9 show that the formulations of the invention are chemically and physically stable at room temperature conditions and therefore no refrigerated storage condition is required thereby promoting an easily drug supply chain and patient compliance with the drug storage recommendations.

### Comparative examples

The following examples disclose the preparation of ophthalmic formulations containing [(2S)-1-(4-{[(3-chloro-4-methoxyphenyl)methyl]amino}-5-{[(pyrimidin-2yl)methyl] carbamoyl}pyrimidin-2-yl)pyrrolidin-2-yl]methyl 6-(nitrooxy)hexanoate (Compound (I)), the vehicles of the ophthalmic formulations of the comparative examples 20 to 24 contain the same "surfactant component" and co-solvent of the formulations of the invention, but the amount of the "surfactant component" and the co-solvent are less than the amounts of the invention. The ophthalmic formulations of comparative examples 25 and 26 contain only a surfactant at a concentration within the range of the invention while the concentration of the co-solvent is higher than the concentration of the co-solvent of the formulation of the invention. The formulations of the comparative examples were prepared by adding 1% w/w of Compound (I) (examples 20-23 and 25) or 1.5% w/w of Compound (I) (examples 24 and 25), at the end of the preparation the macroscopic appearance of the formulations and the amount of Compound (I) dissolved in the vehicle of the formulations were evaluated.

### Example 20

Macrogolglycerol ricinoleate (Kolliphor^{®} EL) 350 g, Macrogol stearate 40 (Myrj^{™} S40) 50 mg, Polyethylene glycol 400 (Kollisolv^{®} PEG E 400) 50 mg, benzalkonium chloride 1 mg and water for injection 9.3 g were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added and the mixture was stirred until complete dissolution. The solution was heated up to 65°C, then Compound (I) 100 mg was added and the mixture was stirred for 30 minutes. The mixture was cooled to room temperature (RT) under stirring. Disodium ethylenediaminetetraacetate dihydrate (EDTA) 10 mg was added and the mixture was stirred for 10 minutes. Water for injection was added to adjust the final weight to 10 g. The pH was 7.6.

The resulting mixture was a suspension. The suspension was centrifuged and the amount of Compound (I) in the supernatant solution (vehicle) was quantified by HPLC against a calibration curve. The formulation was stored at RT, 31 weeks after the preparation a sample of the suspension was centrifuged and the amount of Compound (I) in the supernatant solution was quantified by HPLC against a calibration curve. The results of the two analyses are reported in the table below (Table 10).

| **Table 10: Stability data of formulation of Example 20** | | |
|---|---|---|
| **Time (weeks)** | **t0 (Release)** | **t31** |
| Macroscopic appearance | White, milky suspension | White, milky suspension |
| pH | 7.6 | 7.4 |
| Compound (I) Quantification (%w/w) | 0.6 | 0.5 |
| Compound (I) Purity (area%) | 96.7 | 93.7 |

### Example 21

Macrogolglycerol ricinoleate (Kolliphor^{®} EL) 350 mg, Polyoxyethylenesorbitan monooleate (Tween^{®} 80) 50 mg, Polyethylene glycol 400 (Kollisolv^{®} PEG E 400) 50 mg, benzalkonium chloride 1 mg and water for injection 9.3 g were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added and the mixture was stirred until complete dissolution. The solution was heated up to 65°C, then Compound (I) 100 mg was added and the mixture was stirred for 30 minutes. The mixture was cooled down to room temperature (RT) under stirring. Disodium ethylenediaminetetraacetate dihydrate (EDTA) 10 mg was added and the mixture was stirred for 10 minutes. Water for injection was added to adjust the final weight to 10 g. The pH was 7.6.

The resulting mixture was a suspension. The suspension was centrifuged and the amount of Compound (I) in the supernatant solution (vehicle) was quantified by HPLC against a calibration curve. The formulation was stored at RT, 30 weeks after the preparation a sample of the suspension was centrifuged and the amount of Compound (I) in the supernatant solution was quantified by HPLC against a calibration curve. The results of the two analyses are reported in the table below (Table 11).

| **Table 11: Stability data of formulation of Example 21** | | |
|---|---|---|
| **Time (weeks)** | **t0 (Release)** | **t30** |
| Macroscopic appearance | White, milky suspension | White, milky suspension |
| pH | 7.6 | 7.4 |
| Compound (I) Quantification (%w/w) | 0.6 | 0.5 |
| Compound (I) Purity (area%) | 96.8 | 93.7 |

### Example 22

Macrogolglycerol ricinoleate (Kolliphor^{®} EL) 350 mg, Macrogol stearate 40 (Myrj^{™} S40) 50 mg, Polyethylene glycol 400 (Kollisolv^{®} PEG E 400) 50 mg, benzalkonium chloride 1 mg and water 9 g for injection were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added and the mixture was stirred until complete dissolution. The pH was adjusted to 3.1 - 4.2 by adding HCl 0.5M. The solution was heated up to 65°C, then Compound (I) 100 mg was added and the mixture was stirred for 30 minutes. The mixture was cooled down to room temperature (RT) under stirring. Disodium ethylenediaminetetraacetate dihydrate (EDTA) 10 mg was added and the mixture was stirred for 1 hour. Water for injection was added to adjust the final weight to 10 g. The pH was 4.2.

The resulting mixture was a suspension. The suspension was centrifuged and the amount of Compound (I) in the supernatant solution (vehicle) was quantified by HPLC against a calibration curve. The formulation was stored at RT, 27 weeks after the preparation, a sample of the suspension was centrifuged and the amount of Compound (I) in the supernatant solution was quantified by HPLC against a calibration curve. The results of the two analyses are reported in the table below (Table 12).

| **Table 12: Stability data of formulation of Example 22** | | |
|---|---|---|
| **Time (weeks)** | **t0 (Release)** | **t27** |
| Macroscopic appearance | White, milky suspension | White, milky suspension |
| pH | 4.2 | 4.2 |
| Compound (I) Quantification (%w/w) | 0.8 | 0.5 |
| Compound (I) Purity (area%) | 96.8 | 86.8 |

### Example 23

Macrogolglycerol ricinoleate (Kolliphor^{®} EL) 350 mg, Polyoxyethylenesorbitan monooleate (Tween^{®} 80) 50 mg, of Polyethylene glycol 400 (Kollisolv^{®} PEG E 400) 50 mg, benzalkonium chloride 1 mg and water for injection 9 g were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added; the mixture was stirred until complete dissolution. The pH was adjusted to 3.1 - 4.2 by adding dropwise HCl 0.5M. The solution was heated up to 65°C, then Compound (I) 100 mg was added and the mixture was stirred for 30 minutes. The mixture was cooled down to room temperature (RT) under stirring. Disodium ethylenediaminetetraacetate dihydrate (EDTA) 10 mg was added and the mixture was stirred for 1 hour. Water for injection was added to adjust the final weight to 10 g. The pH was 4.2.

The resulting mixture was a suspension. The suspension was centrifuged and the amount of Compound (I) in the supernatant solution (vehicle) was quantified by HPLC against a calibration curve. The formulation was stored at RT, 27 weeks after the preparation, a sample of the suspension was centrifuged and the amount of Compound (I) in the supernatant solution was quantified by HPLC against a calibration curve. The results of the two analyses are reported in the table below (Table 13).

| **Table 13: Stability data of formulation of Example 23** | | |
|---|---|---|
| **Time (weeks)** | **t0 (Release)** | **t27** |
| Macroscopic appearance | White suspension | White suspension |
| pH | 4.2 | 4.3 |
| Compound (I) Quantification (%w/w) | 0.8 | 0.6 |
| Compound (I) Purity (area%) | 96.9 | 88.8 |

### Example 24

Macrogolglycerol ricinoleate (Kolliphor^{®} EL) 350 mg, Macrogol stearate 40 (Myrj^{™} S40) 50 mg, Polyethylene glycol 400 (Kollisolv^{®} PEG E 400) 550 mg, benzalkonium chloride 1 mg and water for injection 8 g were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added; the mixture was stirred until complete dissolution. The solution was heated up to 65°C, then Compound (I) 150 mg was added and the mixture was and stirred for 30 minutes. The mixture was cooled down to room temperature (RT) under stirring and disodium ethylenediaminetetraacetate dihydrate (EDTA) 10 mg was added and the mixture was stirred for 20 minutes. Water for injection was added to adjust the final weight to 10 g. The pH was 7.6.

The resulting mixture was a suspension. The suspension was centrifuged and the amount of Compound (I) in the supernatant solution (vehicle) was quantified by HPLC against a calibration curve. The formulation was stored at RT, 2 weeks after the preparation, an aliquot was withdrawn, centrifuged and the amount of Compound (I) in the supernatant solution was quantified by HPLC against a calibration curve. The results of the two independent analyses are reported in the table below (Table 14).

| **Table 14: Stability data of formulation of Example 24** | | |
|---|---|---|
| **Time (weeks)** | **t0 (Release)** | **t2** |
| Macroscopic appearance | White, milky suspension | White, milky suspension |
| pH | 7.6 | 7.4 |
| Compound (I) Quantification (%w/w) | 0.6 | 0.5 |
| Compound (I) Purity (area%) | 99.1 | 98.9 |

### Example 25

Macrogol stearate 40 (Myrj^{™} S40) 650 mg, Polyethylene glycol 400 (Kollisolv^{®} PEG E 400) 50 mg, benzalkonium chloride 1 mg and water for injection 9.1 g were weighted into the preparation vessel. The mixture was stirred using a magnetic stir-plate until a slightly yellow or colorless solution was obtained. Boric acid (H₃BO₃) 19 mg and disodium hydrogen phosphate heptahydrate (Na₂HPO₄·7H₂O) 51 mg were added; the mixture was stirred until complete dissolution. The solution was heated up to 65°C, then Compound (I) 100 mg was added and the mixture was stirred for additional 30 minutes. The mixture was cooled down to room temperature (RT) under stirring and disodium ethylenediaminetetraacetate dihydrate (EDTA) 10 mg was added while stirring for 10 more minutes. Water for injection was added to adjust the final weight to 10 g. The pH was 7.6.

The resulting mixture was a suspension. The suspension was centrifuged and the amount of Compound (I) in the supernatant solution (vehicle) was quantified by HPLC against a calibration curve. The formulation was stored at RT, 30 weeks after the preparation an aliquot was withdrawn, centrifuged and the amount of Compound (I) quantified by HPLC against a calibration curve. The results of two independent analyses are reported in the table below (Table 15).

| **Table 15: Stability data of formulation of Example 25** | | |
|---|---|---|
| **Time (weeks)** | **t0 (Release)** | **t30** |
| Macroscopic appearance | White, milky suspension | Clear solution without visible particles |
| pH | 7.6 | 7.4 |
| Compound (I) Quantification (%w/w) | 0.4 | 0.3 |
| Compound (I) Purity (area%) | 96.7 | 92.0 |

### Example 26

Macrogol stearate 40 (Myrj^{™} S40) 650 mg, Polyethylene glycol 400 (Kollisolv^{®} PEG E 400) 550 mg, benzalkonium chloride 1 mg and water for injection 8.5 g were weighted into the preparation vessel. The mixture was stirred using a magnetic stir-plate until obtaining a slightly yellow or colorless solution. Boric acid (H₃BO₃) 19 mg and disodium hydrogen phosphate heptahydrate (Na₂HPO₄·7H₂O) 51 mg were added; the mixture was stirred until complete dissolution. The solution was heated up to 65°C, then Compound (I) 150 mg was added and the mixture was and stirred for 30 minutes. The mixture was cooled down to room temperature (RT) under stirring. Disodium ethylenediaminetetraacetate dihydrate (EDTA) 10 mg was added and the mixture was stirred for 20 minutes. Water for injection was added to adjust the final weight to 10 g. The pH was 7.6.

The resulting mixture was a suspension. The suspension was centrifuged and the amount of Compound (I) in the supernatant solution (vehicle) was quantified by HPLC against a calibration curve. The formulation was stored at RT, 2 weeks after the preparation, a sample of the suspension was centrifuged and the amount of Compound (I) in the supernatant solution was quantified by HPLC against a calibration curve. The results of the two analyses are reported in the table below (Table 16).

| **Table 16: Stability data of formulation of Example 26** | | |
|---|---|---|
| **Time (weeks)** | **t0 (Release)** | **t2** |
| Macroscopic appearance | White, milky suspension | Clear solution without visible particles |
| pH | 7.6 | 7.4 |
| Compound (I) Quantification (%w/w) | 0.6 | 0.6 |
| Compound (I) Purity (area%) | 99.2 | 98.9 |

### Comments

The formulations of the comparative examples contain the same surfactant components and co-solvent of those of the formulations of the invention but at lower concentrations (see examples 20 to 24). Alternatively, other formulations contain the surfactant and co-solvent at higher concentration than that in the formulation of the invention (examples 25 and 26).

All the formulations of the comparative examples were suspensions.

The results of the analyses of the formulations of the comparative examples showed that the concentrations of Compound (I) in the supernatant solution of the formulations of Examples 20, 21, 24 to 26 were below 0.8%. At lower pH (pH 4.2) the concentration of Compound (I) increased up to 0.8%, however, the pH had an impact on the degradation of the active principle, namely after 27 weeks of storage a decrease of about 8 to 10 % purity was observed (see Examples 22 and 23).

A summary of the stability analyses of the comparative examples 20 to 26 and Example 1, that refers to an ophthalmic formulation of the invention containing 1% w/w of Compound (I) according to the invention, is reported in Table 17.

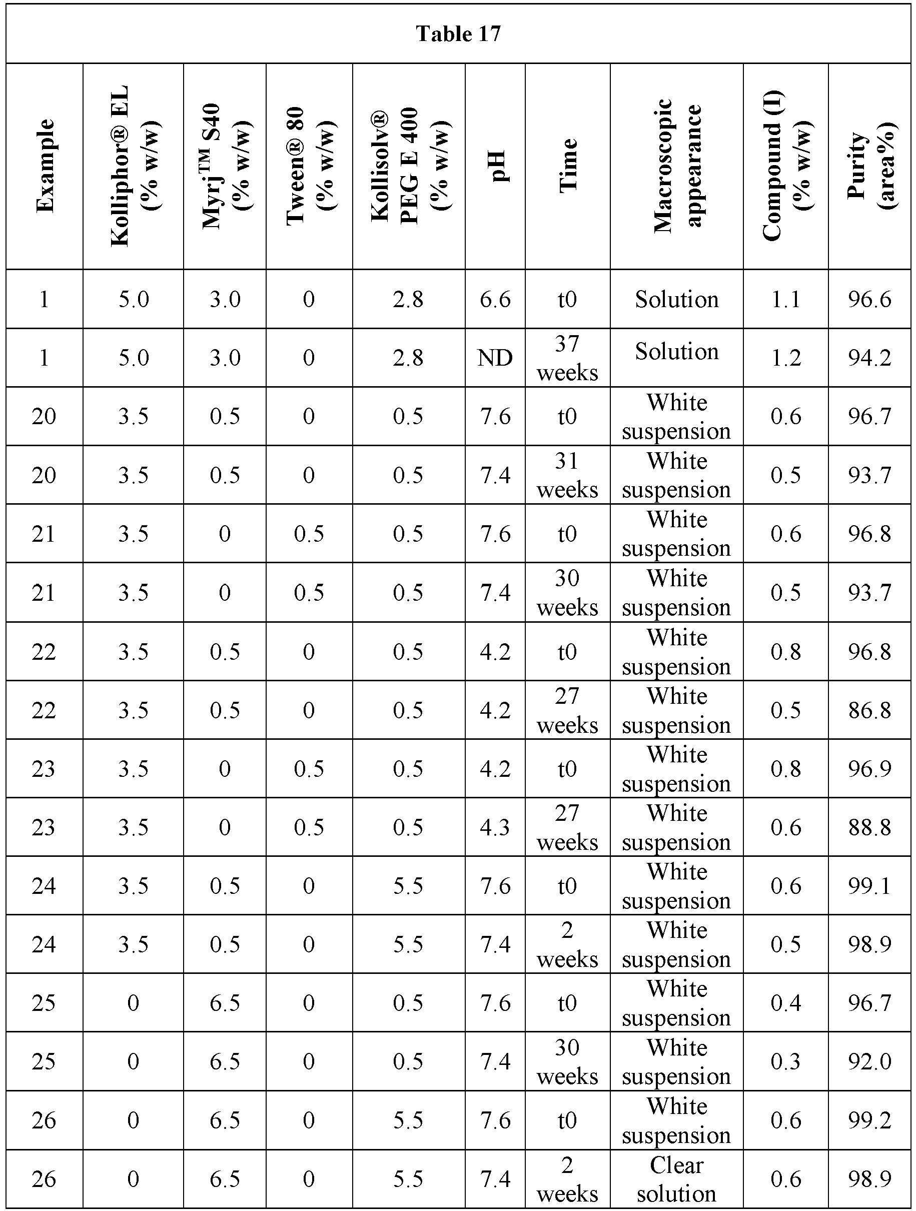

## Claims

1. An ophthalmic formulation comprising:
(i) 0.8% to 1.8% w/w of [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate or any specific value within said range;
(ii) a surfactant component selected from the following mixtures:
- 4.0% to 7.5% w/w Macrogolglycerol ricinoleate and 1.0% to 6.0% w/w Macrogol stearate 40,
- 4.0% to 7.5% w/w Macrogolglycerol ricinoleate and 1.0% to 7.0% w/w Polyoxyethylenesorbitan monooleate, or
- 1.0% to 6.0% w/w Macrogol stearate 40 and 1.0% to 7.0% w/w Polyoxyethylenesorbitan monooleate;
(iii) 1.0% to 5.0% w/w of a co-solvent selected from the class of polyols;
(iv) a buffer system to adjust the pH in a range of 4.5 to 7.5;
(v) water for injection or purified water q.s. to 100% w/w,
and wherein the osmolality of the ophthalmic formulations is 240 to 400 mOsm/kg, preferably 240 to 380 mOsm/kg.

2. The ophthalmic formulation according to claim 1 comprising:
(i) 1.0% to 1.8% w/w of [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate or any specific value within said range;
(ii) a surfactant component selected from the following mixtures:
- 5.0% to 7.5% w/w Macrogolglycerol ricinoleate and 3.0% to 5.0% w/w Macrogol stearate 40,
- 5.0% to 7.5% w/w of Macrogolglycerol ricinoleate and 2.0% to 5.0% w/w Polyoxyethylenesorbitan monooleate, or
- 3.0% to 5.0% w/w Macrogol stearate 40 and 2.0% to 5.0% w/w Polyoxyethylenesorbitan monooleate;
(iii) 1.0% to 4.0% w/w Polyethylene glycol 400;
(iv) a buffer system to adjust the pH to 4.5 to 7.5;
(v) water for injection or purified water q.s. to 100% w/w;
and further comprising 0.01% to 0.10% w/w EDTA and 0.01% to 0.02% w/w benzalkonium chloride.

3. The ophthalmic formulation according to claim 1 comprising:
(i) 1.0% to 1.5% w/w of [(2S)-1-(4-{[(3-chloro-4-methoxyphenyl)methyl]amino}-5-{[(pyrimidin-2-yl)methyl]carbamoyl}-pyrimidin-2-yl)pyrrolidin-2-yl]methyl 6-(nitrooxy)hexanoate or any specific value within said range;
(ii) a surfactant component selected from the following mixtures:
- 5.0% to 7.5% w/w Macrogolglycerol ricinoleate and 3.0% to 5.0% w/w Macrogol stearate 40,
- 5.0% to 7.5% w/w Macrogolglycerol ricinoleate and 2.0% to 5.0% w/w Polyoxyethylenesorbitan monooleate, or
- 3.0% to 5.0% w/w Macrogol stearate 40 and 2.0% to 5.0% w/w Polyoxyethylenesorbitan monooleate;
(iii) 1.0% to 4.0% w/w of a co-solvent selected from the class of polyols;
(iv) a buffer system to adjust the pH over a range of 4.5 to 7.5;
(v) water for injection or purified water q.s. to 100% w/w.

4. The ophthalmic formulation according to any of claims 1 to 3 wherein the co-solvent is selected from mannitol, glycerol, sorbitol or polyethylene glycols.

5. The ophthalmic composition according to any of claims 1 to 3 wherein the co-solvent is Polyethylene glycol 400.

6. The ophthalmic formulation according to any of claims 1 to 5 wherein the buffer system is selected from the following: boric acid and disodium hydrogen phosphate heptahydrate, disodium hydrogen phosphate heptahydrate and sodium dihydrogen phosphate dihydrate, disodium hydrogen phosphate heptahydrate and citric acid, trisodium citrate dihydrate and citric acid monohydrate, trisodium citrate dihydrate and boric acid, boric acid.

7. The ophthalmic formulation according to any of claims 1 to 6 wherein the buffer system is selected from boric acid and disodium hydrogen phosphate heptahydrate or citric acid monohydrate and trisodium citrate dehydrate or trisodium citrate dihydrate and boric acid or boric acid.

8. The ophthalmic composition according to any of claims 1 to 7 further comprises a chelating agent.

9. The ophthalmic formulation according to claim 8 wherein the chelating agent is 0.01% to 0.2% w/w ethylenediaminetetraacetic acid or its salts.

10. The ophthalmic formulation according to any of claims 1 to 9 further comprising an antimicrobial preservative selected from benzalkonium chloride, polyquaternium-1, benzethonium chloride, potassium sorbate or sorbic acid or a mixture thereof.

11. The ophthalmic formulation according to claim 10 wherein the antimicrobial preservative is benzalkonium chloride.

12. An ophthalmic formulation according to any of claims 1 to 11 further comprising one or more tonicity adjusting agents selected from: glycerol, sorbitol, mannitol, dextrose, sodium or potassium chloride.

13. An ophthalmic formulation according to any of claims 1 to 12 further comprising a viscosity enhancing agent selected from methyl cellulose, hydroxypropyl methylcellulose, antioxidants or a mixture thereof.

14. An ophthalmic formulation according to claim 1 consisting essentially of:
(i) 1.0% w/w [(2S)-1-(4-{[(3-chloro-4-methoxyphenyl)methyl]amino}-5-{[(pyrimidin-2-yl)methyl] carbamoyl}pyrimidin-2-yl)pyrrolidin-2-yl]methyl 6-(nitrooxy)hexanoate;
(ii) 5.0% w/w Macrogolglycerol ricinoleate and 3.0% w/w Macrogol stearate 40;
(iii) 2.8% w/w Polyethylene glycol 400;
(iv) 0.19% w/w boric acid, 0.51% w/w disodium hydrogen phosphate heptahydrate;
(v) water for injection q.s. to 100% w/w;
and further comprising 0.1% w/w disodium ethylenediaminetetraacetate dihydrate, 0.01% w/w benzalkonium chloride and HCl or NaOH to adjust the pH to 6.5 - 6.9.

15. An ophthalmic formulation according to claim 1 consisting essentially of:
(i) 1.3% w/w [(2S)-1-(4-{[(3-chloro-4-methoxyphenyl)methyl]amino}-5-{[(pyrimidin-2-yl)methyl] carbamoyl}pyrimidin-2-yl)pyrrolidin-2-yl]methyl 6-(nitrooxy)hexanoate;
(ii) 7.5% w/w Macrogolglycerol ricinoleate and 5.0% w/w Macrogol stearate 40;
(iii) 2.8% w/w Polyethylene glycol 400;
(iv) 0.19% w/w boric acid, 0.51% w/w disodium hydrogen phosphate heptahydrate;
(v) water for injection q.s. to 100% w/w;
and further comprising 0.1% w/w disodium ethylenediaminetetraacetate dihydrate, 0.01% w/w benzalkonium chloride, and HCl or NaOH to adjust pH to 6.5 - 6.9.

16. An ophthalmic formulation according to claim 1 consisting essentially of:
(i) 1.4% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate;
(ii) 7.5% w/w Macrogolglycerol ricinoleate and 5.0% w/w Macrogol stearate 40;
(iii) 4.0% w/w Polyethylene glycol 400;
(iv) 0.19% w/w boric acid, 0.51% w/w disodium hydrogen phosphate heptahydrate;
(v) water for injection q.s. to 100% w/w;
and further comprising 0.1% w/w disodium ethylenediaminetetraacetate dihydrate, 0.01% w/w benzalkonium chloride, and HCl to adjust pH to 6.5 - 6.9.

17. An ophthalmic formulation according to claim 1 consisting essentially of:
(i) 1.8% w/w [(2S)-1-(4-{[(3-chloro-4-methoxyphenyl)methyl]amino}-5-{[(pyrimidin-2-yl)methyl] carbamoyl}pyrimidin-2-yl)pyrrolidin-2-yl]methyl 6-(nitrooxy)hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.51% w/w disodium hydrogen phosphate heptahydrate;
(v) water for injection q.s. to 100% w/w;
and further comprising 0.1% w/w disodium ethylenediaminetetraacetate dihydrate, 0.01% w/w benzalkonium chloride, and HCl or NaOH to adjust pH to 6.5 - 6.9.

18. An ophthalmic formulation according to claim 1 consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.51% w/w disodium hydrogen phosphate heptahydrate;
(v) water for injection or purified water q.s. to 100% w/w;
and further comprising 0.1% w/w disodium ethylenediaminetetraacetate dihydrate, 0.01% w/w benzalkonium chloride.

19. An ophthalmic formulation according to claim 1 consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate and 0.73% w/w trisodium citrate dihydrate;
(v) water for injection or purified water q.s. 100% w/w;
and further comprising 0.1% w/w disodium ethylenediaminetetraacetate dihydrate, 0.01% w/w benzalkonium chloride.

20. An ophthalmic formulation according to claim 1 consisting of essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.19% w/w boric acid, 0.003% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

21. An ophthalmic formulation according to claim 1 consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate, 0.73% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

22. An ophthalmic formulation according to claim 1 consisting essentially of:
(i) 1.7% w/w [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy) hexanoate;
(ii) 7.5% w/w Macrogolglycerol ricinoleate and 5.0% w/w Polyoxyethylenesorbitan monooleate;
(iii) 1.0% w/w Polyethylene glycol 400;
(iv) 0.11% w/w citric acid monohydrate, 0.73% w/w trisodium citrate dihydrate;
(v) water for injection or purified water q.s. to 100% w/w;
and further comprising 0.1% w/w disodium ethylenediaminetetraacetate dihydrate, 0.01% w/w benzalkonium chloride.

23. An ophthalmic formulation according to any of claims 1 to 19 for use for treating glaucoma, ocular hypertension, pathological conditions associated with elevated intraocular pressure or retinopathies.

## Patentansprüche

1. Ophthalmologische Formulierung, umfassend:
(i) 0,8 bis 1,8 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat oder einen beliebigen spezifischen Wert innerhalb dieses Bereichs;
(ii) eine Tensidkomponente, ausgewählt aus den folgenden Mischungen:
- 4,0 bis 7,5 Gew.-% Macrogolglycerolricinoleat und 1,0 bis 6,0 Gew.-% Macrogolstearat 40,
- 4,0 bis 7,5 Gew.-% Macrogolglycerolricinoleat und 1,0 bis 7,0 Gew.-% Polyoxyethylensorbitanmonooleat oder
- 1,0 bis 6,0 Gew.-% Macrogolstearat 40 und 1,0 bis 7,0 Gew.-% Polyoxyethylensorbitanmonooleat;
(iii) 1,0 bis 5,0 Gew.-% eines aus der Klasse der Polyole ausgewählten Co-Lösungsmittels;
(iv) ein Puffersystem zur Einstellung des pH-Werts in einem Bereich von 4,5 bis 7,5;
(v) Wasser für Injektionszwecke oder gereinigtes Wasser q.s. bis 100 Gew.-%,
wobei die Osmolalität der ophthalmischen Formulierungen 240 bis 400 mOsm/kg, vorzugsweise 240 bis 380 mOsm/kg, beträgt.

2. Ophthalmologische Formulierung nach Anspruch 1, umfassend:
(i) 1,0 bis 1,8 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat oder einen beliebigen spezifischen Wert innerhalb des genannten Bereichs;
(ii) eine Tensidkomponente, ausgewählt aus den folgenden Mischungen:
- 5,0 bis 7,5 Gew.-% Macrogolglycerolricinoleat und 3,0 bis 5,0 Gew.-% Macrogolstearat 40,
- 5,0 bis 7,5 Gew.-% Macrogolglycerolricinoleat und 2,0 bis 5,0 Gew.-% Polyoxyethylensorbitanmonooleat, oder
- 3,0 bis 5,0 Gew.-% Macrogolstearat 40 und 2,0 bis 5,0 Gew.-% Polyoxyethylensorbitanmonooleat;
(iii) 1,0 bis 4,0 Gew.-% Polyethylenglykol 400;
(iv) ein Puffersystem zur Einstellung des pH-Werts auf 4,5 bis 7,5;
(v) Wasser für Injektionszwecke oder gereinigtes Wasser q.s. bis 100 Gew.-%;
und ferner umfassend 0,01 bis 0,10 Gew.-% EDTA und 0,01 bis 0,02 Gew.-% Benzalkoniumchlorid.

3. Ophthalmologische Formulierung nach Anspruch 1, umfassend:
(i) 1,0 bis 1,5 Gew.-% [(2S)-1-(4-{[(3-Chlor-4-methoxyphenyl)methyl]amino}-5-{[(pyrimidin-2-yl)methyl]carbamoyl}-pyrimidin-2-yl)pyrrolidin-2-yl]methyl-6-(nitrooxy)hexanoat oder einen beliebigen spezifischen Wert innerhalb des genannten Bereichs;
(ii) eine Tensidkomponente, ausgewählt aus den folgenden Mischungen:
- 5,0 bis 7,5 Gew.-% Macrogolglycerolricinoleat und 3,0 bis 5,0 Gew.-% Macrogolstearat 40,
- 5,0 bis 7,5 Gew.-% Macrogolglycerolricinoleat und 2,0 bis 5,0 Gew.-% Polyoxyethylensorbitanmonooleat, oder
- 3,0 bis 5,0 Gew.-% Macrogolstearat 40 und 2,0 bis 5,0 Gew.-% Polyoxyethylensorbitanmonooleat;
(iii) 1,0 bis 4,0 Gew.-% eines Co-Lösungsmittels, ausgewählt aus der Klasse der Polyole;
(iv) ein Puffersystem zur Einstellung des pH-Werts in einem Bereich von 4,5 bis 7,5;
(v) Wasser für Injektionszwecke oder gereinigtes Wasser q.s. bis 100 Gew.-%.

4. Ophthalmologische Formulierung nach einem der Ansprüche 1 bis 3, wobei das Co-Lösungsmittel ausgewählt ist aus Mannit, Glycerin, Sorbit oder Polyethylenglykolen.

5. Ophthalmologische Zusammensetzung nach einem der Ansprüche 1 bis 3, wobei das Co-Lösungsmittel Polyethylenglykol 400 ist.

6. Ophthalmologische Formulierung nach einem der Ansprüche 1 bis 5, wobei das Puffersystem ausgewählt ist aus: Borsäure und Dinatriumhydrogenphosphat-Heptahydrat, Dinatriumhydrogenphosphat-Heptahydrat und Natriumdihydrogenphosphat-Dihydrat, Dinatriumhydrogenphosphat-Heptahydrat und Zitronensäure, Trinatriumcitrat-Dihydrat und Zitronensäure-Monohydrat, Trinatriumcitrat-Dihydrat und Borsäure, Borsäure.

7. Ophthalmologische Formulierung nach einem der Ansprüche 1 bis 6, wobei das Puffersystem ausgewählt ist aus Borsäure und Dinatriumhydrogenphosphat-Heptahydrat oder Zitronensäure-Monohydrat und Trinatriumcitrat-Dehydrat oder Trinatriumcitrat-Dihydrat und Borsäure oder Borsäure.

8. Ophthalmologische Zusammensetzung nach einem der Ansprüche 1 bis 7, ferner umfassend einen Chelatbildner.

9. Ophthalmologische Formulierung nach Anspruch 8, wobei der Chelatbildner 0,01 bis 0,2 Gew.-% Ethylendiamintetraessigsäure ist oder deren Salze.

10. Ophthalmologische Formulierung nach einem der Ansprüche 1 bis 9, ferner umfassend ein antimikrobielles Konservierungsmittel, ausgewählt aus Benzalkoniumchlorid, Polyquaternium-1, Benzethoniumchlorid, Kaliumsorbat oder Sorbinsäure oder einer Mischung davon.

11. Ophthalmologische Formulierung nach Anspruch 10, wobei das antimikrobielle Konservierungsmittel Benzalkoniumchlorid ist.

12. Ophthalmologische Formulierung nach einem der Ansprüche 1 bis 11, ferner umfassend ein oder mehrere tonizitätsregulierende Mittel, ausgewählt aus: Glycerin, Sorbit, Mannit, Dextrose, Natrium- oder Kaliumchlorid.

13. Ophthalmologische Formulierung nach einem der Ansprüche 1 bis 12, ferner umfassend ein viskositätserhöhendes Mittel, ausgewählt aus Methylcellulose, Hydroxypropylmethylcellulose, Antioxidantien oder einer Mischung davon.

14. Ophthalmologische Formulierung nach Anspruch 1, im Wesentlichen bestehend aus:
(i) 1,0 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 5,0 Gew.-% Macrogolglycerolricinoleat und 3,0 Gew.-% Macrogolstearat 40;
(iii) 2,8 Gew.-% Polyethylenglykol 400;
(iv) 0,19 Gew.-% Borsäure, 0,51 Gew.-% Dinatriumhydrogenphosphat-Heptahydrat;
(v) Wasser für Injektionszwecke q.s. bis 100 Gew.-%;
und ferner umfassend 0,1 Gew.-% Dinatriumethylendiamintetraacetat-Dihydrat, 0,01 Gew.-% Benzalkoniumchlorid und HCl oder NaOH, um den pH-Wert auf 6,5 bis 6,9 einzustellen.

15. Ophthalmologische Formulierung nach Anspruch 1, im Wesentlichen bestehend aus:
(i) 1,3 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Macrogolstearat 40;
(iii) 2,8 Gew.-% Polyethylenglykol 400;
(iv) 0,19 Gew.-% Borsäure, 0,51 Gew.-% Dinatriumhydrogenphosphat-Heptahydrat;
(v) Wasser für Injektionszwecke q.s. bis 100 Gew.-%;
und ferner umfassend 0,1 Gew.-% Dinatriumethylendiamintetraacetat-Dihydrat, 0,01 Gew.-% Benzalkoniumchlorid und HCl oder NaOH zur Einstellung des pH-Werts auf 6,5 bis 6,9.

16. Ophthalmologische Formulierung nach Anspruch 1, im Wesentlichen bestehend aus:
(i) 1,4 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Macrogolstearat 40;
(iii) 4,0 Gew.-% Polyethylenglykol 400;
(iv) 0,19 Gew.-% Borsäure, 0,51 Gew.-% Dinatriumhydrogenphosphat-Heptahydrat;
(v) Wasser für Injektionszwecke q.s. bis 100 Gew.-%;
und ferner umfassend 0,1 Gew.-% Dinatriumethylendiamintetraacetat-Dihydrat, 0,01 Gew.-% Benzalkoniumchlorid und HCl zur Einstellung des pH-Werts auf 6,5 - 6,9.

17. Ophthalmologische Formulierung nach Anspruch 1, im Wesentlichen bestehend aus:
(i) 1,8 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,19 Gew.-% Borsäure, 0,51 Gew.-% Dinatriumhydrogenphosphat-Heptahydrat;
(v) Wasser für Injektionszwecke q.s. bis 100 Gew.-%;
und ferner umfassend 0,1 Gew.-% Dinatriumethylendiamintetraacetat-Dihydrat, 0,01 Gew.-% Benzalkoniumchlorid und HCl oder NaOH zur Einstellung des pH-Werts auf 6,5 bis 6,9.

18. Ophthalmologische Formulierung nach Anspruch 1, im Wesentlichen bestehend aus:
(i) 1,7 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,19 Gew.-% Borsäure, 0,51 Gew.-% Dinatriumhydrogenphosphat-Heptahydrat;
(v) Wasser für Injektionszwecke oder gereinigtes Wasser q.s. bis 100 Gew.-%;
und ferner umfassend 0,1 Gew.-% Dinatriumethylendiamintetraacetat-Dihydrat, 0,01 Gew.-% Benzalkoniumchlorid.

19. Ophthalmologische Formulierung nach Anspruch 1, im Wesentlichen bestehend aus:
(i) 1,7 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,11 Gew.-% Zitronensäure-Monohydrat und 0,73 Gew.-% Trinatriumcitrat-Dihydrat;
(v) Wasser für Injektionszwecke oder gereinigtes Wasser q.s. bis 100 Gew.-%;
und ferner umfassend 0,1 Gew.-% Dinatriumethylendiamintetraacetat-Dihydrat, 0,01 Gew.-% Benzalkoniumchlorid.

20. Ophthalmologische Formulierung nach Anspruch 1, im Wesentlichen bestehend aus:
(i) 1,7 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,19 Gew.-% Borsäure, 0,003 Gew.-% Trinatriumcitrat-Dihydrat;
(v) Wasser für Injektionszwecke oder gereinigtes Wasser q.s. bis 100 Gew.-%;
und ferner umfassend 0,1 Gew.-% Dinatriumethylendiamintetraacetat-Dihydrat, 0,01 Gew.-% Benzalkoniumchlorid.

21. Ophthalmologische Formulierung nach Anspruch 1, im Wesentlichen bestehend aus:
(i) 1,7 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,11 Gew.-% Zitronensäure-Monohydrat, 0,73 Gew.-% Trinatriumcitrat-Dihydrat;
(v) Wasser für Injektionszwecke oder gereinigtes Wasser q.s. bis 100 Gew.-%;
und ferner umfassend 0,1 Gew.-% Dinatriumethylendiamintetraacetat-Dihydrat, 0,01 Gew.-% Benzalkoniumchlorid.

22. Ophthalmologische Formulierung nach Anspruch 1, im Wesentlichen bestehend aus:
(i) 1,7 Gew.-% [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat;
(ii) 7,5 Gew.-% Macrogolglycerolricinoleat und 5,0 Gew.-% Polyoxyethylensorbitanmonooleat;
(iii) 1,0 Gew.-% Polyethylenglykol 400;
(iv) 0,11 Gew.-% Zitronensäure-Monohydrat, 0,73 Gew.-% Trinatriumcitrat-Dihydrat;
(v) Wasser für Injektionszwecke oder gereinigtes Wasser q.s. bis 100 Gew.-%;
und ferner umfassend 0,1 Gew.-% Dinatriumethylendiamintetraacetat-Dihydrat, 0,01 Gew.-% Benzalkoniumchlorid.

23. Ophthalmologische Formulierung nach einem der Ansprüche 1 bis 19 zur Verwendung bei der Behandlung von Glaukom, okulärer Hypertension, pathologischen Zuständen im Zusammenhang mit erhöhtem Augeninnendruck oder Retinopathien.

## Revendications

1. Formulation ophtalmique comprenant :
(i) 0,8 % à 1,8 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ou toute valeur spécifique comprise dans ladite plage ;
(ii) un constituant tensioactif choisi parmi les mélanges suivants :
- 4,0 % à 7,5 % p/p de ricinoléate de macrogolglycérol et 1,0 % à 6,0 % p/p de stéarate de macrogol 40,
- 4,0 % à 7,5 % p/p de ricinoléate de macrogolglycérol et 1,0 % à 7,0 % p/p de monooléate de polyoxyéthylènesorbitane, ou
- 1,0 % à 6,0 % p/p de stéarate de macrogol 40 et 1,0 % à 7,0 % p/p de monooléate de polyoxyéthylènesorbitane ;
(iii) 1,0 % à 5,0 % p/p d'un cosolvant choisi dans la classe des polyols ;
(iv) un système tampon pour ajuster le pH dans une plage de 4,5 à 7,5 ;
(v) de l'eau pour préparations injectables ou de l'eau purifiée q.s. à 100 % p/p,
et dans laquelle l'osmolalité des formulations ophtalmiques est de 240 à 400 mOsm/kg, de préférence de 240 à 380 mOsm/kg.

2. Formulation ophtalmique selon la revendication 1, comprenant :
(i) 1,0 % à 1,8 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ou toute valeur spécifique comprise dans ladite plage ;
(ii) un constituant tensioactif choisi parmi les mélanges suivants :
- 5,0 % à 7,5 % p/p de ricinoléate de macrogolglycérol et 3,0 % à 5,0 % p/p de stéarate de macrogol 40,
- 5,0 % à 7,5 % p/p de ricinoléate de macrogolglycérol et 2,0 % à 5,0 % p/p de monooléate de polyoxyéthylènesorbitane, ou
- 3,0 % à 5,0 % p/p de stéarate de macrogol 40 et 2,0 % à 5,0 % p/p de monooléate de polyoxyéthylènesorbitane ;
(iii) 1,0 % à 4,0 % p/p de polyéthylène glycol 400 ;
(iv) (un système tampon pour ajuster le pH entre 4,5 et 7,5 ;
(v) de l'eau pour préparations injectables ou de l'eau purifiée q.s. à 100 % p/p ;
et comprenant en outre 0,01 % à 0,10 % p/p d'EDTA et 0,01 % à 0,02 % p/p de chlorure de benzalkonium.

3. Formulation ophtalmique selon la revendication 1, comprenant :
(i) 1,0 % à 1,5 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ou toute valeur spécifique comprise dans ladite plage ;
(ii) un constituant tensioactif choisi parmi les mélanges suivants :
- 5,0 % à 7,5 % p/p de ricinoléate de macrogolglycérol et 3,0 % à 5,0 % p/p de stéarate de macrogol 40,
- 5,0 % à 7,5 % p/p de ricinoléate de macrogolglycérol et 2,0 % à 5,0 % p/p de monooléate de polyoxyéthylènesorbitane, ou
- 3,0 % à 5,0 % p/p de stéarate de macrogol 40 et 2,0 % à 5,0 % p/p de monooléate de polyoxyéthylènesorbitane ;
(vi) 1,0 % à 4,0 % p/p d'un cosolvant choisi dans la classe des polyols ;
(vii) un système tampon pour ajuster le pH sur une plage de 4,5 à 7,5 ;
(viii) de l'eau pour préparations injectables ou de l'eau purifiée q.s. à 100 % p/p.

4. Formulation ophtalmique selon l'une quelconque des revendications 1 à 3, dans laquelle le cosolvant est choisi parmi le mannitol, le glycérol, le sorbitol ou les polyéthylène glycols.

5. Composition ophtalmique selon l'une quelconque des revendications 1 à 3, dans laquelle le cosolvant est le polyéthylène glycol 400.

6. Formulation ophtalmique selon l'une quelconque des revendications 1 à 5, dans laquelle le système tampon est choisi parmi les suivants : l'acide borique et l'hydrogénophosphate de disodium heptahydraté, l'hydrogénophosphate de disodium heptahydraté et le dihydrogénophosphate de sodium dihydraté, l'hydrogénophosphate de disodium heptahydraté et l'acide citrique, le citrate trisodique dihydraté et l'acide citrique monohydraté, le citrate trisodique dihydraté et l'acide borique, l'acide borique.

7. Formulation ophtalmique selon l'une quelconque des revendications 1 à 6, dans laquelle le système tampon est choisi parmi l'acide borique et l'hydrogénophosphate de sodium heptahydraté, ou l'acide citrique monohydraté et le citrate trisodique dihydraté, ou le citrate trisodique dihydraté et l'acide borique ou l'acide borique.

8. Composition ophtalmique selon l'une quelconque des revendications 1 à 7, comprenant en outre un agent chélateur.

9. Formulation ophtalmique selon la revendication 8, dans laquelle l'agent chélateur est 0,01 % à 0,2 % p/p d'acide éthylènediaminetétraacétique ou de ses sels.

10. Formulation ophtalmique selon l'une quelconque des revendications 1 à 9, comprenant en outre un conservateur antimicrobien choisi parmi le chlorure de benzalkonium, le polyquaternium-1, le chlorure de benzéthonium, le sorbate de potassium ou l'acide sorbique ou un mélange de ceux-ci.

11. Formulation ophtalmique selon la revendication 10, dans laquelle le conservateur antimicrobien est le chlorure de benzalkonium.

12. Formulation ophtalmique selon l'une quelconque des revendications 1 à 11, comprenant en outre un ou plusieurs agents d'ajustement de la tonicité choisis parmi : le glycérol, le sorbitol, le mannitol, le dextrose, le chlorure de sodium ou de potassium.

13. Formulation ophtalmique selon l'une quelconque des revendications 1 à 12, comprenant en outre un agent augmentant la viscosité choisi parmi la méthylcellulose, l'hydroxypropylméthylcellulose, des antioxydants ou un mélange de ceux-ci.

14. Formulation ophtalmique selon la revendication 1, constituée essentiellement de :
(i) 1,0 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 5,0 % p/p de ricinoléate de macrogolglycérol et 3,0 % p/p de stéarate de macrogol 40 ;
(iii) 2,8 % p/p de polyéthylène glycol 400 ;
(iv) 0,19 % p/p d'acide borique, 0,51 % p/p d'hydrogénophosphate de disodium heptahydraté ;
(v) de l'eau pour préparations injectables q.s. à 100 % p/p ;
et comprenant en outre 0,1 % p/p d'éthylènediaminetétraacétate de disodium dihydraté, 0,01 % p/p de chlorure de benzalkonium, et de l'HCl ou du NaOH pour ajuster le pH à 6,5-6,9.

15. Formulation ophtalmique selon la revendication 1, constituée essentiellement de :
(i) 1,3 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de stéarate de macrogol 40 ;
(iii) 2,8 % p/p de polyéthylène glycol 400 ;
(iv) 0,19 % p/p d'acide borique, 0,51 % p/p d'hydrogénophosphate de disodium heptahydraté ;
(v) de l'eau pour préparations injectables q.s. à 100 % p/p ;
et comprenant en outre 0,1 % p/p d'éthylènediaminetétraacétate de disodium dihydraté, 0,01 % p/p de chlorure de benzalkonium, et de l'HCl ou du NaOH pour ajuster le pH à 6,5-6,9.

16. Formulation ophtalmique selon la revendication 1, constituée essentiellement de :
(i) 1,4 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de stéarate de macrogol 40 ;
(iii) 4,0 % p/p de polyéthylène glycol 400 ;
(iv) 0,19 % p/p d'acide borique, 0,51 % p/p d'hydrogénophosphate de disodium heptahydraté ;
(v) de l'eau pour préparations injectables q.s. à 100 % p/p ;
et comprenant en outre 0,1 % p/p d'éthylènediaminetétraacétate de disodium dihydraté, 0,01 % p/p de chlorure de benzalkonium, et de l'HCl pour ajuster le pH à 6,5-6,9.

17. Formulation ophtalmique selon la revendication 1, constituée essentiellement de :
(i) 1,8 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,19 % p/p d'acide borique, 0,51 % p/p d'hydrogénophosphate de disodium heptahydraté ;
(v) de l'eau pour préparations injectables q.s. à 100 % p/p ;
et comprenant en outre 0,1 % p/p d'éthylènediaminetétraacétate de disodium dihydraté, 0,01 % p/p de chlorure de benzalkonium, et de l'HCl ou du NaOH pour ajuster le pH à 6,5 - 6,9.

18. Formulation ophtalmique selon la revendication 1, constituée essentiellement de :
(i) 1,7 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,19 % p/p d'acide borique, 0,51 % p/p d'hydrogénophosphate de disodium heptahydraté ;
(v) de l'eau pour préparations injectables ou de l'eau purifiée q.s. à 100 % p/p ;
et comprenant en outre 0,1 % p/p d'éthylènediaminetétraacétate de disodium dihydraté, 0,01 % p/p de chlorure de benzalkonium.

19. Formulation ophtalmique selon la revendication 1, constituée essentiellement de :
(i) 1,7 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,11 % p/p d'acide citrique monohydraté et 0,73 % p/p de citrate trisodique dihydraté ;
(v) de l'eau pour préparations injectables ou de l'eau purifiée q.s. 100 % p/p ;
et comprenant en outre 0,1 % p/p d'éthylènediaminetétraacétate de disodium dihydraté, 0,01 % p/p de chlorure de benzalkonium.

20. Formulation ophtalmique selon la revendication 1, constituée essentiellement de :
(i) 1,7 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,19 % p/p d'acide borique, 0,003 % p/p de citrate trisodique dihydraté ;
(v) de l'eau pour préparations injectables ou de l'eau purifiée q.s. à 100 % p/p ;
et comprenant en outre 0,1 % p/p d'éthylènediaminetétraacétate de disodium dihydraté, 0,01 % p/p de chlorure de benzalkonium.

21. Formulation ophtalmique selon la revendication 1, constituée essentiellement de :
(i) 1,7 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,11 % p/p d'acide citrique monohydraté, 0,73 % p/p de citrate trisodique dihydraté ;
(v) de l'eau pour préparations injectables ou de l'eau purifiée q.s. à 100 % p/p ;
et comprenant en outre 0,1 % p/p d'éthylènediaminetétraacétate de disodium dihydraté, 0,01 % p/p de chlorure de benzalkonium.

22. Formulation ophtalmique selon la revendication 1, constituée essentiellement de :
(i) 1,7 % p/p de 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle ;
(ii) 7,5 % p/p de ricinoléate de macrogolglycérol et 5,0 % p/p de monooléate de polyoxyéthylènesorbitane ;
(iii) 1,0 % p/p de polyéthylène glycol 400 ;
(iv) 0,11 % p/p d'acide citrique monohydraté, 0,73 % p/p de citrate trisodique dihydraté ;
(v) de l'eau pour préparations injectables ou de l'eau purifiée q.s. à 100 % p/p ;
et comprenant en outre 0,1 % p/p d'éthylènediaminetétraacétate de disodium dihydraté, 0,01 % p/p de chlorure de benzalkonium.

23. Formulation ophtalmique selon l'une quelconque des revendications 1 à 19, pour une utilisation pour traiter le glaucome, l'hypertension oculaire, les états pathologiques associés à une pression intraoculaire élevée ou les rétinopathies.
